# EUROPÄISCHE PATENTANMELDUNG

(11) **EP 2 465 567 A1**
(43) Veröffentlichungstag der Anmeldung: **20.06.2012**
(21) Anmeldenummer: 11194351.0
(22) Anmeldetag: 19.12.2011
(51) Int. Cl.: A61M 21/00, A61N 5/06, F21S 10/00, F21V 9/10

(54) **Verwendung optischer Signale zur Beeinflussung, Stimulation und Korrektion der menschlichen Psyche und Steigerung des Wohlbefindens**

(30) Priorität: 17.12.2010 EP 10195596
(71) Anmelder: Zaitchik, Semen, 10365 Berlin (DE)
(72) Erfinder: Zaitchik, Semen, 10365 Berlin (DE)
(74) Vertreter: Lange, Sven

(57) **Zusammenfassung**

Die Erfindung beschreibt ein Verfahren zur Beeinflussung eines physiologischen und/oder psychologischen Vorgangs, bei dem optische Signale auf einer Projektionsfläche rhythmisch und sinuswellenartig wiedergegeben werden, wobei die Änderung der optischen Signale subsensorisch (unbewusst) mit einer definierten Frequenz erfolgt.

## Beschreibung

Die Erfindung betrifft ein Verfahren physiologischen und/oder psychologischen Vorgangs, wobei optische Signale auf eine Projektionsfläche wiedergegeben werden, und die optischen Signale rhythmisch und sinuswellenartig geändert werden, wobei die Signale subsensorisch wirken.

Im Stand der Technik sind Verfahren bekannt, die allgemein als Bioresonanztherapie bezeichnet werden und zur Behandlung von Allergien, Migräne, Schlafstörungen, chronischen Schmerzen und weiteren Krankheiten verwendet werden. Bei der Bioresonanz beziehungsweise den entsprechenden Geräten, werden Elektroden mit mindestens zwei Stellen der Haut eines Probanden in Verbindung gebracht. Im einfachsten Fall nimmt der Proband zwei bewegliche Elektroden in jeweils eine Hand. Die elektrischen Signale interferieren mit körpereigenen elektrischen Signalen, wodurch ggf. krankhafte elektromagnetische Schwingungen aufgehoben werden können. Dies hat unter Umständen einen positiven Effekt auf eine Krankheit des Probanden.

Ein ähnlicher Effekt kann bei der Anwendung von Edelsteinen, Metallen und Farbkarten beobachtet werden, die auch über heilsame Schwingungen verfügen.

Im Stand der Technik ist die Chronophysiologie bekannt. Die Chronophysiologie beschäftigt sich mit der zeitlichen Organisation von Lebewesen. Sie berücksichtigt dabei psychologische Rhythmen, die sich in Verbindung mit dem Faktor Zeit verändern. Ein psychologischer Rhythmus kann beispielsweise für einen bestimmten Menschen festlegen, zu welchen Tageszeiten er die beste Aufmerksamkeit zum Lernen hat, oder welcher Schiaf-Wach-Rhythmus sich optimal auf sein Wohlbefinden auswirkt. Es ist beispielsweise möglich, ein Profil seiner täglichen Leistungsfähigkeit zu erstellen. Dazu werden physiologische Körperfunktionen, mentale Kriterien (wie Aufmerksamkeit, intellektuelle Leistungsfähigkeit) und deren zeitliche Veränderung erfasst. Chronopsychologie ist eine Erweiterung der Chronobiologie und der Chronomedizin. Dabei werden "äußere Einflüsse" wirksam, wie Schichtarbeit, Zeitumstellung durch Flugreisen (sog. Jet-Lag), Sommerzeit/Winterzeit. Ebenso werden "innere Einflüsse" wirksam, wie biologische Rhythmen, altersbedingte Veränderungen, oder ob der Mensch eher ein Morgen- oder Abendtyp (Chronotypen) ist. Psychologische Rhythmen eines Menschen werden beeinflusst durch die Situation, die Tätigkeit und durch individuelle Faktoren des einzelnen Menschen. Die Biorhythmologie ist ebenfalls eine Wissenschaft, die im Stand der Technik beschrieben ist und sich mit dem Biorhythmus befasst. Der Biorhythmus ist eine Erscheinung bei Organismen, der bestimmte Lebensvorgänge in einer festen Periodisierung ablaufen lässt. Man spricht dann auch von einer biologischen oder biochemischen Oszillation. Die Vorgänge müssen selbsterregt (endogen) sein, um von einem Biorhythmus sprechen zu können. Er bildet beim Menschen, bei den Tieren und den Pflanzen die Grundlage für die Chronobiologie. Die Periodizität bzw. Phasen sind bei einzelnen Organismen von unterschiedlicher Dauer. Jeder Organismus richtet sich nach dem Tagesrhythmus, der durch die Erddrehung bestimmt wird. Doch haben Untersuchungen gezeigt, dass die innere Uhr des Menschen nur etwa 23 Stunden für einen Tag anzeigt. Andere Perioden werden z.B. durch Ebbe und Flut (Gezeiten; je 12,8 Stunden) bestimmt, durch die Mondphase mit 28 Tagen (lunar) oder 14-15 Tagen (semi-lunar), durch die Jahreszeiten (unter anderem Winterschlaf bei Tieren, Vogelzug, Blattabwurf bei Pflanzen, Eisprung, Wanderung der Aale und Lachse) und durch klimatische Faktoren, die sehr unterschiedlich sein können. Einige Periodizitäten konnten bisher aber noch nicht gedeutet werden.

Im Stand der Technik sind Verfahren offenbart, die der Beeinflussung des Biorhythmus des Menschen dienen. So beschreibt beispielsweise die DE 1 954 929 7 C2 ein Verfahren und eine Vorrichtung zur Beeinflussung der menschlichen Psyche. Hierbei wird die Augenbewegung einer Person elektrisch im Augenbereich durch Sensoren erfasst und die Messwerte an ein Auswertungssystem übertragen. Das Auswertungssystem bewertet hiernach den psychischen Zustand der Person und steuert ein Stimulationssystem, was in Abhängigkeit der Bewertung des psychischen Zustands der Person Frequenz und/oder Amplituden modulierte akustische Grundmuster oder Farbsignale als Stimulationssignale appliziert.

Durch solche Verfahren soll die psychische Belastung, die durch erhöhte berufliche und private Beanspruchung hervorgerufen wird, kompensiert werden. Die psychische Belastung, die im alltäglichen Sprachgebrauch als Stress bezeichnet wird, beeinflusst nicht nur die Leistungsfähigkeit einer Person, sondern auch den Gesundheitszustand dieser.

Um die psychische Belastung einer Person zu kompensieren, ist weiterhin in der DE 102 004 031 459 A1 ein Verfahren beschrieben, bei der eine Person ein Bild für eine Entspannung vorgelegt wird. Das Bild ändert sich ständig, wobei die Geschwindigkeit der Veränderung des Bildes so eingestellt wird, dass der Betrachter diese Veränderung bei ständiger Betrachtung des Bildes nicht feststellt. Das Bild erscheint der Person in jedem einzelnen Augeblick als nicht verändert. Hierdurch soll eine Entspannung erreicht werden, die für Therapie- und Übungszwecke verwendet werden kann. Im Stand der Technik ist beschrieben, dass die minimale Geschwindigkeit einer fließenden Bewegung von Objekten auf einem Hintergrund aus anderen Objekten, bei der diese Bewegung von einem Betrachter noch wahrgenommen werden kann, ein bis zwei Winkelsekunden pro Sekunde beträgt.

Weiterhin beschreibt die DE 1 996 156 8 A1 ein Verfahren zum Steigern des psychischen und physischen Wohlbefindens und zur Therapie bestimmter Krankheitsbilder. Das Verfahren wird vorzugsweise in Kombination mit einer Sauerstofftherapie angewendet, bei der Sauerstoff mittels einer Atemmaske inhaliert wird. Dem Anwender werden hierbei audiovisuelle Eindrücke mittels einem Kopfhörer und einer speziellen Brille, die beide mit einer Mindmaschine verbunden sind, übermittelt. Die Sauerstofftherapie wird angewandt, um bei bekannten Krankheitsbildern eine Verbesserung zu erzielen. Außerdem kann die Konzentrations- und Leistungsfähigkeit sowie die Minderung von Stresssymptomen erzielt werden. Der einzuatmende Sauerstoff ist vorzugsweise ionisiert, d.h. mit negativen Ionen angereichert. Während der Sauerstofftherapie trägt der Anwender einen Kopfhörer und eine Brille, die beide mit einer sogenannten mind-machine verbunden sind. In diesem Gerät werden die Impulse und Signale erzeugt, die in dem Kopfhörer und der Brille zu den akustischen und optischen Reizen und Eindrücken umgewandelt werden. Der Anwender erfährt somit eine Sauerstofftherapie und gleichzeitig erlebt er die Eindrücke einer mind-machine, die zu einer mentalen Entspannung führt.

Für die Anwendung der im Stand der Technik beschriebenen Verfahren und Methoden muss ein Anwender aktiv an dem Verfahren teilnehmen und es bedarf eines hohen apparativen und organisationellen Aufwandes. Nachteilig ist außerdem, dass keine Stimulation über einen langen Zeitraum möglich ist, da die Belastung für die Person zu hoch ist. Außerdem ist eine impulsartige Stimulation mittels einer mind-machine nicht oder nur schwer mit einem Rhythmus eines Organismus zu vereinbaren, da es sich bei der impulsartigen Stimulation um keinen natürlich vorkommenden Rhythmus handelt. Weiterhin muss der Patient oder Proband an spezielle Geräte oder Apparaturen angeschlossen werden, was die Verwendbarkeit und die Anwendungsmöglichkeiten erheblich einschränkt.

Aufgabe der Erfindung war es demgemäß, ein Verfahren bereitzustellen, was nicht die Nachteile oder Mängel des Standes der Technik aufweist.

Gelöst wird die Aufgabe durch die unabhängigen Ansprüche, vorteilhafte Ausführungsformen finden sich in den abhängigen Ansprüchen.

Es war völlig überraschend dass ein Verfahren zur Beeinflussung eines physiologischen und/oder psychologischen Vorgangs bereitgestellt werden kann, was nicht die Nachteile oder Mängel des Standes der Technik aufweist, wobei das Verfahren ein optische Signale wiedergebendes Leuchtmittel umfasst, das bevorzugt so ausgerichtet ist, dass ein Patient oder ein Anwender die optischen Signale unbewusst erfährt und wobei die optischen Signale rhythmisch und sinuswellenartig ändernde Signale sind, und die Änderung der Signale innerhalb von 6 Sekunden bis 12 Stunden, bevorzugt 6 Sekunden bis 1 Stunde, besonders bevorzugt 10 Sekunden bis 15 Minuten, erfolgt. Die Änderungen erfolgen insbesondere subsensorisch.

Es war völlig überraschend, dass die Änderungen der Signale so langsam geschehen, dass ein Mensch die Änderungen in einem normalen Zustand nicht bemerken kann. Es handelt sich bevorzugt um eine subsensorische Wahrnehmung. Die Erfindung eröffnet somit einen neuen technischen Bereich, da im Stand der Technik lediglich Verfahren mit Biofeedback beschrieben sind, bei denen die Patienten durch optische Signale beeinflusst werden, wobei die optischen Signale bemerkbar sind. In einem Bereich von 6 Sekunden bis 12 Stunden kann sich ein optisches Signal sehr langsam entfalten, so dass die Änderung für einen Betrachter unbemerkt bleibt.

In einer besonders bevorzugten Ausführungsform erfolgt die Änderung mit einer Frequenz von 6 Sekunden bis 1 Stunde, was insbesondere einen Effekt auf Atmung, hypothalamische, nebennierenrindische und physiologische Aktivität sowie Eiweißstoffsynthese und Zellmembran Durchlässigkeit aufweist.

Vorteilhafterweise kann das Verfahren zur subsensorischen Stimulation eines Patienten oder eines Betrachters verwendet werden. Die bevorzugt unbewusste Wahrnehmung ermöglicht die Nutzung ohne speziell organisierte Prozeduren beim alltäglichen Tagesverlauf, was einen erheblichen Vorteil gegenüber den im Stand der Technik offenbarten Verfahren und Techniken darstellt. Ein weiterer Vorteil der Erfindung ist, dass nur bei der unbewussten Wahrnehmung die laengeren Rhythmen beeinfluesst werden, weil für das Ergebniss viel Zeit in Anspruch genommen werden muss (z.B. in Fall Dekaminutenrhythmen braucht man mind. 30 Wiederholungen und dass 4 mal pro Tag). So kann vorteilhafterweise ein Patient oder Anwender während der Arbeit, während einer Freizeitbeschäftigung, beim Schlafen, beim Fernsehen oder bei der Arbeit am PC, auf einem Handy, Smartphone, Tablet-Rechner, das erfindungsgemäße Verfahren verwenden. Und auch bei den Rhythmen, die in Form von Beleuchtungsänderungen (Lampen) organisiert sind.

Die Projektionsfläche, auf die das Leuchtmittel einwirkt, kann bevorzugt den vollständigen Bildschirm einnehmen, wobei es auch vorteilhaft sein kann, lediglich einen Teil dieser zu verwenden. Vorteilhafterweise befindet sich die Projektionsfläche im Sichtfeld des Anwenders und kann auch im Standby Modus genutzt werden. Das Leuchtmittel ist bevorzugt ein Display, ein Monitor, oder eine Lampe, wobei der Monitor, bzw. der Display ein Bestandteil eines elektronischen Gerätes wie ein Fernseher, Tablet, Handy, Smartphone, Computer oder MP3-Wiedergabegerät sein kann. Vorteilhafterweise kann jeder Display als Leuchtmitel genutzt werden, wodurch das Verfahren universell einsetzbar und verwendbar ist. Der Anwender kann bevorzugt auch über einen längeren Zeitraum das Verfahren nutzen, ohne dass dies für ihn körperlich oder psychisch belastend wäre. Vorteilhafterweise können auch alle Arten von Projektionsebenen wie beispielsweise Leinwand, Tapete Decke oder Wand als Projektionsfläche genutzt werden.

Die optischen Signale sind bevorzugt ausgewählt aus der Gruppe umfassend gegenständliche Abbildungen, Farben, Farbabbildungen oder Projektionen unterschiedlicher Lichtintensitäten. Die Änderung der Signale, insbesondere der optischen Signale erfolgt sinuswellenartig. Es war völlig überraschend, dass sinuswellenartige optische Signale einen Einfluss auf den menschlichen Organismus, insbesondere Organe Psyche und mentale Zustaende haben. Die Erfindung betrifft auch die Verwendung sinuswellenartiger optischer Signale zur Beeinflussung elektromagnetischer Schwingungen, biochemischer, hormoneller oder andere Prozesse in einem Körper und Psyche. Die rhythmische Aktivität eines Organismus zeigt sich vorteilhafterweise auf allen Ebenen. Es ist jedoch bevorzugt, dass die rhythmischen Prozesse mit einer Frequenz oder Intervall von mehreren Sekunden bis zu mehreren Stunden mit der hormonellen/humoralen Regulation und trophischen Prozessen verbunden sind.

Es ist bevorzugt, dass mehrere optische Signale mit unterschiedlichen Rhythmen dargestellt werden und vorteilhafterweise eine Änderung der Signale für einen Betrachter nicht bemerkbar sind. Das heißt insbesondere, dass ein Betrachter die Änderung der optischen Signale, insbesondere der Abbildungen oder Farben nicht bewusst wahrnimmt, da diese innerhalb von 6 Sekunden bis 12 Stunden, bevorzugt 6 Sekunden bis 1 Stunde, besonders bevorzugt 10 Sekunden bis 15 Minuten, geändert werden und die Änderungen für einen Betrachter nicht wahrnehmbar ablaufen. Das hat wesentliche Vorteile gegenüber dem Stand der Technik, da das bevorzugte Verfahren von einem Betrachter "nebenbei" betrachtet werden kann. Beispielsweise kann das Verfahren auf einem Handydisplay oder Fernsehapparat oder einer Lampe ausgeführt werden, so dass der Betrachter seiner Tätigkeit nachgehen kann, aber die langsame Änderung der optischen Signale eine positive Wirkung auf z. B. sein Wohlbefinden hat. Diesbezüglich kann es vorteilhaft sein, wenn das Leuchtmittel eine Lampe ist, die über einen längeren Zeitraum das Licht ändert.

Es war völlig überraschend, dass Rhythmen mit einer Frequenz ab 6 bis 30 Sekunden einen Effekt auf Atmungsprozesse und sympatische/parasympatische Aktivitäten oder motorische Aktivitäten zeigen. Es ist bevorzugt, dass mehrere optische Signale mit unterschiedlichen Rhythmen dargestellt werden, so dass die Rhythmen überlagert werden und ggf. Interferenzen entstehen. Weiterhin ist es vorteilhaft, dass Dekasekunden und Minuten Rhythmen beschreiben, die elektrische Aktivität der Hirnrinde zeigen und die Periodik der emotionalen Zustände und Temperaturegulation beeinflussen. Rhythmen mit einer Frequenz von Stunden beeinflussen vorzugsweise den Hypothalamus und die Nierenrinde und damit auch alle psychische und physiologische Prozesse die von den entsprechenden Hormone reguliert.

Die Projektionsfläche ist bevorzugt ausgewählt aus der Gruppe umfassend ein Display, ein Monitor, eine Tapete und eine Leinwand. Im Sinne der Erfindung können alle elektronischen Geräte, die eine Projektionsfläche aufweisen, von dem Verfahren verwendet werden. Solche elektronischen Geräte umfassen außerdem Handys, MP3-Player, Laptops, Computer, Smartphones, Fernsehgeräte usw. Das Verfahren kann hierbei vorteilhafterweise mittels einem Speichermedium auf den entsprechenden elektronischen Geräten wiedergegeben werden. Der Fachmann weiß, dass Speichermedien beispielsweise CDs, Micro-Discs, Flash-Speicher oder sonstige magnetische Speichermedien umfasst.

Der Benutzer kann beispielsweise das Verfahren auf seinem Handy oder seinem MP3-Player installieren und es sich während der Fahrt zur Arbeit ansehen oder auf sich optisch wirken lassen. Das Verfahren beeinflusst insbesondere das menschliche psychologische und physiologische Verhalten, wobei dieses stimuliert, entspannt und korrigiert wird. Es kann weiterhin für Übungs-, Lern-, Meditations-, und Werbungszwecke verwendet werden. Hierbei wird der bewusste Gedankenablauf verlangsamt, was wiederum zur Vertiefung des Denkens führt und Grundlage für alle Meditationstechniken ist. Versuche haben weiterhin gezeigt, dass hierdurch eine effektive Art zum Lernen und des intellektuellen Trainings bereitgestellt werden kann. Außerdem können mit dem erfindungsgemäßen Verfahren die tranceähnlichen Zustände der Psyche erreicht werden. Weiterhin hat sich gezeigt, dass das erfindungsgemäße Verfahren großflächig auf Tapeten oder Wänden darstellen lässt und als Verzierung verwendet wird. Es können in Abhängigkeit der Tageszeit- oder Jahreszeit unterschiedliche Bilder als exogene Zeitgeber angezeigt werden.

Es ist bevorzugt, dass sich die Farbe, der Ton, die Farbdichte/Farbsättigung, die Helligkeit und/oder die Intensität der Signale ändern Außerdem kann sich vorteilhafterweise auch der Inhalt eines Bildes ändern. Die Änderung kann beispielsweise eine Bewegung von einem oder mehrerer Punkte oder filmartige Änderungen, wie z. B. ein Sonnenaufgang am Strand umfassen.

Es ist so eine einfache und schnelle Anpassung möglich, wobei die minimale wahrnehmbare Unterscheidungsstufe von jedem Parameter für das Sehvermögen insbesondere 1% der ursprünglichen Größe beträgt.

Die Rhythmen können vorteilhafterweise in ihrer Frequenz geändert werden. Außerdem kann die Superposition der Rhythmen verändert werden, so dass mehrere Rhythmen überlagert werden. Es ist auch bevorzugt, die bestimmte endogene Rhythmen in bestimmten Tageszeiten anzuwenden und einen Einfluss auf endogene Rhythmen zu erreichen, um hierdurch einen stimulierenden und/oder entspannenden Effekt auf einen Betrachter oder Anwender auszuüben. Die Rhythmen können bevorzugt in endogene oder exogene Rhythmen unterteilt werden. Die Phasenänderung der Rhythmen (d. h. ob sich die Akrophase verkürzt oder verlängert), kann ebenfalls als bevorzugte Ausführungsform verwendet werden.

Es ist demgemäß bevorzugt, dass die mehreren optischen Signale mit unterschiedlichen Rhythmen hintereinander gezeigt werden Es kann auch bevorzugt sein, mehrere Rhythmen aufeinander zu legen (Superposition) oder die gegebenen Rhythmen bezüglich innerer Rhythmen des Organismus zu verschieben, um vorteilhafterweise eine Resonanz oder Interferenz zu erreichen. Die optischen Signale können vorteilhafterweise durch alle künstlichen Lichtquellen erzeugt werden. Hierzu zählen unter anderem Lampen, Beleuchtung von elektronischen Geräten, Ambilight bei Fernsehgeräten Projektoren oder sonstige Lichtquellen. Die optischen Signale sind vorteilhafterweise exogene Zeitgeber hier besser sagen Rhythmusgeber. Exogene Zeitgeber auch Rhythmusgeber bezeichnen im Sinne der Erfindung insbesondere alle im Tagesablauf schwankenden Umweltbedingungen (bspw. Licht, Temperatur, oder Luftfeuchtigkeit). Als exogene Zeitgeber können jedoch auch soziale Zeitgeber, wie z.B. das Verhalten der Artgenossen bezeichnet werden. Die exogenen oder äußeren Zeitgeber werden vorteilhafterweise von den Augen aufgenommen und zur Zirbeldrüse weitergeleitet. Diese produziert tagesrhythmisch das Hormon Melatonin, das den Wach- Schlafrhythmus beeinflusst. Als endogene Zeitgeber wird vorzugsweise die sogenannte innere Uhr bezeichnet, die in jeder Zelle die gesamten Stoffwechselprozesse in Abhängigkeit von der Zeit (Tages-, Schlafrhythmus) beeinflusst. Im Energiestoffwechsel der Zelle können periodische Zyklen beobachtet werden, die vermutlich die Grundlage für die zeitliche Steuerung für Entwicklung und Verhalten von Personen darstellen.

Es bevorzugt, dass die Wahrnehmung der rhythmischen sinuswellenartigen Änderungen bei dauerhafter Anwendung zu einer Phasenverschiebung eines endogenen Vorgangs führt. Mittelwellige Rhythmen, deren Perioden Minuten oder Stunden dauern, können bei Organen, Organsystemen oder Systemen der inneren Sekretion aber auch bei Vorgängen wie Verdauung, Transport von rhythmischer Verteilung der Nährstoffe oder die rhythmische Freisetzung von Hormonen festgestellt werden. Es war völlig überraschend, dass das erfindungsgemäße Verfahren, insbesondere die sinuswellenartigen Änderungen, endogene Vorgänge beeinflussen können.

Die minimalen wahrnehmbaren Unterscheidungsstufen von jedem Parameter (insbesondere Farbsättigung, Farbintensität und Helligkeit) für das Sehvermögen betragen einen hundertstel Teil von der ursprünglichen Größe. Die minimalen räumlichen Unterscheidungsmöglichkeiten betragen eine Winkelminute und die minimalen Unterscheidungsmöglichkeiten für die Bewegung sind zwei Winkelsekunden pro Sekunde. Es kann auch bevorzugt sein, dass die Farbabbildung ihre Farbe mit einer Geschwindigkeit von weniger als 5 Nanometer pro Sekunde ändern.

Es hat sich überraschenderweise herausgestellt, dass eine Verwendung des erfindungsgemäßen Verfahrens zur Entspannung, Abbau von Ermüdungserscheinungen und/oder Verbesserung der Konzentration oder mentaler Zustände führt. Im wesentlichen kann das bevorzugte Verfahren zur Entspannung genutzt werden.

Es war überraschend, dass Rhythmen oder optische Signale, die sich in Perioden ab 6 Sekunden bis 12 Stunden ändern menschliche Prozesse im Köper beeinflussen können. Allgemein sind diese Prozesse von biochemischer Aktivität (Produktion und Kreislauf von Hormonen, Fermenten, Mediatoren, usw.) und Prozesse von organischen Änderungen in Zellen und Geweben. Das bevorzugte Verfahren ist nicht als Therapieverfahren anzusehen, sondern übt einen beruhigenden oder entspannenden Effekt auf den Körper aus, ohne etwas zu therapieren oder mit dem Körper in irgendeiner Weise verbunden zu sein. Der bevorzugte Frequenzbereich gliedert sich in mehrere Unterbereiche, nämlich:
- Die Frequenzen von 0,15 Hz bis 0,004 Hz (Periode ab 6 bis 25 Sekunden), Änderung in sympatischer und/oder parasympatischer Regulation.
- Rhythmen mit einer Periode von Dekasekunden. Diese zeigen Aktivitätsmuster langregulierenden Hirnsystem, das sich im Hypothalamus und der limbischen Struktur befindet. Dieses System reguliert die Produktion von Mediatoren und ist mit Adaptation- und Stressprozessen beschäftigt.

- Rhythmen mit Minutenperiode charakterisieren den psycho-emotionalen Zustand des Menschen und zeigen die Aktivität der Muskeln und beeinflussen die Herzschlagfrequenz und Atemfrequenz.
- Rhythmen mit Dekaminutenperiode regulieren verschiedene (langsame und schnelle) Schlafphasen, auch die Prozesse von Aufmerksamkeit und motorischer Aktivität.
- Infrastundische Rhythmen (weniger als eine Stunde) zeigen die Prozesse in Zellenniveau, z. B. Proteinsynthese, Änderung in Größe und Masse einer Zelle.
- Ultradianische Rhythmen (mehrere Stunden) charakterisieren die Regulation von Wahrnehmungsprozessen und Prozessen im Gedächtnis und motorische Reflexe.

Es war völlig überraschend, dass die genannten Perioden mit visueller subsensorischer (nichtbewusste) rhythmische Stimulation beeinflusst werden kann. Das bevorzugte Verfahren hat zahlreiche Vorteile gegenüber dem Stand der Technik, z. B. uneingeschränkte Anwendung rund um die Uhr, da das Verfahren jederzeit und an jedem Ort angewendet werden kann. Außerdem können unterschiedliche elektronische Geräte (wie z. B. Lampe, Birne, Displays) für das Verfahren verwendet werden. Überraschenderweise können auch Geräte wie Handys, Smartphones, Tablets, Laptops, Projektionsgeräte, Lampen, Fernsehgeräte, Displays oder MP3-Wiedergabegeräte verwendet werden. Es kann im Wesentlichen jedes Gerät verwendet werden, das einen Monitor oder Display aufweist. Die Leuchtmittel können vorteilhafterweise als Dekoration eingesetzt werden (z. B. als Tapete oder bildliche Uhrzeiger) und finden zudem Verwendung in Meditations- Relaxitions- und Lernzwecken, sowie im Werbe- und Marketingbereich.

In einem weiteren Aspekt betrifft die Erfindung eine Vorrichtung zur Durchführung des Verfahrens, umfassend ein Leuchtmittel und eine Computer-Einheit. Die oben dargestellten Ausführungen und Ausführungsformen gelten analog für die Vorrichtung. Die Vorrichtung umfasst vorzugsweise ein Leuchtmittel, das beispielsweise eine Lampe, ein Display oder eine Projektionsfläche sein kann. Weiterhin umfasst die Vorrichtung bevorzugt eine Computer-Einheit, die die Änderung des Leuchtmittels vornimmt und auch entsprechend programmiert ist. Computer-Einheiten sind insbesondere Bestandteil elektronischer Geräte, ausgewählt aus der Gruppe umfassend Laptop, Computer, Handy, Smartphone, Tablet, Fernseher oder MP3-Wedergabegerät. Die bevorzugte Vorrichtung kann zur subsensorischen optischen Stimulation eines Betrachters verwendet werden. Außerdem kann die Vorrichtung zur Entspannung, Abbau von Ermüdungserscheinungen und/oder Verbesserung der Konzentration verwendet werden.

In diesem Sinne betrifft die Erfindung auch ein Computerprogrammprodukt, mit dem das Verfahren ausführbar ist. Das Computerprogrammprodukt ist vorzugsweise auf einer Computer-Einheit installiert und bewirkt einen technischen Effekt auf das elektrisch bzw. elektronische Gerät, so dass eine Änderung der optischen Signale eintritt. Die Änderungen sind für einen Betrachter vorzugsweise nicht bemerkbar. Es kann jedoch auch bevorzugt sein, dass dem Betrachter erst optische Signale präsentiert werden, deren Änderung er bemerkt und während der Betrachtung die optischen Signale in der Form geändert werden, dass deren Änderung für den Betrachter nicht feststellbar ist.

Im Folgenden soll die Erfindung anhand eines Beispiels erläutert werden, ohne jedoch auf das Beispiel begrenzt zu sein.

### Beispiel 1

Ein Patient oder ein Proband schaut sich auf einer Projektionsfläche einen Film an, der die Änderung einer Farbe (z. B. blau) darstellt. Der Farbton variiert innerhalb einer Zeit von 15, 30, 45 oder 60 Sekunden. Die Farbe kann sich beispielsweise von hell nach dunkel verändern. Der Proband fühlte nach der Betrachtung einen Entspannungszustand.

### Beisiel 2

Drei Patienten oder Probanden haben innerhalb von 10 Minuten einen Film angesehen, wobei sich der Farbton von von hell- bis dunkelblau subsensorisch geändert hat (20 Zycklen pro Film). Eine Kontrollgruppe von ebenfalls drei Patienten betrachtete den gleichen Film, bei dem die Änderungen bemerkbar waren. Der Entspannungseffekt der Probanden, die den Film mit den subsensorischen Änderungen betrachteten war deutlich stärker, als bei der Kontrollgruppe.

### Beispiel 3

Ein Patient oder Proband betrachtet auf einer Projektionsfläche einen Film, der einen Sonnenaufgang am Strand zeigt. Der Film dauert circa 3-4 Minuten. Zu dem Film können akustische Signale abgespielt werden, die dem Betrachter vermitteln, er würde sich an einem Strand befinden. Die Signale können Wassergeräusche, Vögel oder Menschenstimmen umfassen. Der Proband fühlte sich nach der Betrachtung des Films erheblich entspannt.

### Beispiel 4

14 Teilnehmer (männlich/weiblich) im Alter zwischen 25 bis 50 Jahren wurden 5 Minuten dem erfindungsgemäßen Verfahren ausgesetzt. Das heißt, die Teilnehmer betrachteten eine Projektionsfläche, auf der unbemerkbare Änderungen stattfanden. Zusätzlich wurden die Teilnehmer einem Kontrollverfahren ausgesetzt, bei dem sie 5 Minuten lang ein Bild mit sichtbaren Änderungen auf einer Projektionsfläche betrachteten. Zwischen den Intervallen beider Verfahren lag mindestens eine Zeitspanne von 4 Stunden. Nach den Verfahren mussten die Teilnehmer den Entspannungseffekt als stark, mäßig oder unbemerkbar einschätzen. Folgendes Ergebnis konnte erzielt werden:

| | Starker Effekt | Mäßiger Effekt | Kein Effekt |
|---|---|---|---|
| Kontrollverfahren | 35% | 50% | 14% |
| Verfahren | 57% | 42% | 0% |

Das Ergebnis zeigt, dass 57% der Teilnehmer durch das erfindungsgemäße Verfahren einen starken Entspannungseffekt verspürten. Dahingegen beurteilten lediglich 35% der Teilnehmer das Kontrollverfahren als stark entspannend.

## Patentansprüche

1. Verfahren zur Beeinflussung eines physiologischen und/oder psychologischen Vorgangs umfassend ein optische Signale wiedergebendes Leuchtmittel, wobei die optischen Signale rhythmisch und sinuswellenartig ändernde Signale sind, und die Änderung der Signale innerhalb von 6 Sekunden bis 12 Stunden, bevorzugt 6 Sekunden bis 1 Stunde, besonders bevorzugt 10 Sekunden bis 15 Minuten, erfolgt..

2. Verfahren nach Anspruch 1, wobei das Leuchtmittel ausgewählt ist aus der Gruppe umfassend ein Display, ein Monitor oder eine Lampe.

3. Verfahren nach Anspruch 1 oder 2, wobei die optischen Signale Abbildungen, Bilder oder Farbabbildungen umfassen.

4. Verfahren nach mindestens einem der vorhergehenden Ansprüche, wobei das Leuchtmittel auf eine Projektionsfläche abgebildet wird.

5. Verfahren nach mindestens einem der vorhergehenden Ansprüche, wobei mehrere optische Signale mit unterschiedlichen Rhythmen dargestellt werden.

6. Verfahren nach mindestens einem der vorhergehenden Ansprüche, wobei eine Änderung der Signale für einen Betrachter nicht bemerkbar sind.

7. Verfahren nach mindestens einem der vorhergehenden Ansprüche, wobei die optischen Signale exogener Rhythmus und Zeitgeber sind.

8. Verfahren nach mindestens einem der vorhergehenden Ansprüche, wobei die Wahrnehmung der rhythmischen sinuswellenartigen Änderungen bei dauerhafter Anwendung zu einer Phasenverschiebung eines endogenen Vorgangs führt.

9. Verfahren nach mindestens einem der vorhergehenden Ansprüche, wobei die Abbildungen sich mit einer Geschwindigkeit von weniger als 2, bevorzugt weniger als 1 Winkelsekunde pro Sekunde ändern und/oder die Farbabbildungen ihre Farbe mit einer Geschwindigkeit von weniger als 5 nm pro Sekunde ändern.

10. Vorrichtung zur Durchführung des Verfahrens nach den Ansprüchen 1 bis 9, umfassend ein Leuchtmittel und eine Computer-Einheit.

11. Verwendung der Vorrichtung nach Anspruch 10 zur subsensorischen Stimulation eines Betrachters.

12. Verwendung der Vorrichtung nach mindestens einem der vorhergehenden Ansprüche, zur Entspannung, Abbau von Ermüdungserscheinungen und/oder Verbesserung der Konzentration und mentale Zustände.

13. Computerprogrammprodukt zur Durchführung des Verfahrens nach den Ansprüchen 1 bis 9.
